# EUROPÄISCHE PATENTSCHRIFT

(11) **EP 3 365 048 B1**
(45) Veröffentlichungstag und Bekanntmachung des Hinweises auf die Patenterteilung: **28.08.2019**
(21) Anmeldenummer: 16809628.7
(22) Anmeldetag: 12.10.2016
(51) Int. Cl.: A61M 5/20, A61M 5/315, A61M 5/32

(54) **INJEKTIONSGERÄT**
INJECTION DEVICE
DISPOSITIF D'INJECTION

(30) Priorität: 22.10.2015 DE 202015007351 U
(43) Veröffentlichungstag der Anmeldung: 29.08.2018
(73) Patentinhaber: Haselmeier AG, 9001 St. Gallen (CH)
(72) Erfinder: KEITEL, Joachim, 73728 Esslingen (DE)
(74) Vertreter: Reinhardt, Annette
(86) Internationale Anmeldenummer: PCT/EP2016/001696
(87) Internationale Veröffentlichungsnummer: WO 2017/067646

(56) Entgegenhaltungen:
- EP-A2- 1 968 670
- WO-A1-2010/115670
- DE-A1- 19 519 147
- US-A- 4 194 505

## Beschreibung

Die Erfindung betrifft ein Injektionsgerät zum automatischen Auspressen von Injektionsflüssigkeit aus einem Behälter.

Injektionsgeräte zum automatischen Auspressen von Injektionsflüssigkeit aus einem Behälter sind grundsätzlich bekannt. Automatische Injektionsgeräte besitzen üblicherweise eine Feder, die das Auspressen der Injektionsflüssigkeit aus dem Behälter bewirkt. Derartige Injektionsgeräte sind beispielsweise Autoinjektoren, die zum einmaligen Auspressen einer Dosis von Injektionsflüssigkeit aus einer Spritze dienen. Bei derartigen Autoinjektoren wird die gesamte Injektionsflüssigkeit bei einem einzigen Injektionsvorgang ausgepresst. Für unterschiedliche zu verabreichende Dosen von Injektionsflüssigkeit sind unterschiedliche Injektionsgeräte zu wählen.

Aus der WO 2013/117332 A1 ist ein manuell zu betätigendes Injektionsgerät bekannt, bei dem die auszupressende Menge an Injektionsflüssigkeit vom Bediener vor der Injektion einzustellen ist. Dabei wird eine Injektionshülse einen vorgegebenen Weg von einem Anschlag in distale Richtung verstellt. Beim Auspressen von Injektionsflüssigkeit drückt der Bediener das Bedienelement und verschiebt dadurch die Injektionshülse in proximale Richtung, bis diese den Anschlag erreicht. In Abhängigkeit der auszupressenden Menge an Injektionsflüssigkeit ergeben sich dadurch unterschiedliche Startpositionen für die Injektionshülse. Die Endlage der Injektionshülse, die mit der Endlage eines Dosierkolbens, der die Injektionsflüssigkeit auspresst, korrespondiert, ist fest vorgegeben.

Die EP 1 968 670 offenbart einen Autoinjektor mit einer Einstellvorrichtung zur Einstellung der auszupressenden Menge an Injektionsflüssigkeit. Die Einstellvorrichtung umfasst einen Anschlag, dessen axiale Lage über ein Gewinde einstellbar ist.

Aus der DE 195 19 147 A1 geht ein Injektionsgerät hervor, bei dem die auszupressende Dosis durch Längsverstellung eines Gehäuseteils relativ zu einem anderen Gehäuseteil einstellbar ist.

Die US 4,194,505 zeigt ein Injektionsgerät, bei dem die auszupressende Menge an Injektionsflüssigkeit über eine drehbare Kappe einstellbar ist. Die Position der drehbaren Kappe bestimmt die Länge des Kolbenhubs.

Die WO 2010/115670 zeigt ein Injektionsgerät mit einer Einstellvorrichtung, mit der die Lage von zwei Gehäuseteilen zueinander einstellbar ist. Hierüber ist die Menge der auszupressenden Menge an Injektionsflüssigkeit einstellbar.

Der Erfindung liegt die Aufgabe zugrunde, ein Injektionsgerät zum automatischen Auspressen von Injektionsflüssigkeit aus einem Behälter zu schaffen, das einen einfachen Aufbau besitzt und eine Einstellung der auszupressenden Menge an Injektionsflüssigkeit erlaubt.

Es ist vorgesehen, dass das Injektionsgerät eine Einstellvorrichtung zur Einstellung der auszupressenden Menge an Injektionsflüssigkeit besitzt. Das Injektionsgerät besitzt einen Endanschlag, der die Endlage des Dosierkolbens festlegt. Die Endlage des Dosierkolbens kann dabei direkt am Dosierkolben selbst oder indirekt über mit dem Dosierkolben gekoppelten Bauteile erfolgen. Ein einfacher Aufbau des Injektionsgeräts wird dadurch erreicht, dass die Einstellvorrichtung Mittel zur Verstellung der Lage des Endanschlags besitzt. Über die Einstellvorrichtung kann demnach die Endlage des Dosierkolbens eingestellt werden. Die Startposition des Dosierkolbens ist für jede auszupressende Menge an Injektionsflüssigkeit gleich. Dadurch muss der Bediener bei der Einstellung den Dosierkolben nicht bewegen, und die Injektionsfeder kann bereits vorgespannt im Gehäuse des Injektionsgeräts angeordnet sein. Dies ist insbesondere bei hochviskosen Injektionsflüssigkeiten vorteilhaft, die eine sehr stark ausgelegte Injektionsfeder benötigen. Die Injektionsfeder muss dadurch nicht vom Bediener gespannt werden, sondern wird bei der Herstellung des Injektionsgeräts gespannt. Dadurch, dass die Einstellvorrichtung auf die Lage des Endanschlags wirkt, ist dennoch eine Einstellung der auszupressenden Menge an Injektionsflüssigkeit möglich. Bei dem Injektionsgerät handelt es sich vorteilhaft um ein Injektionsgerät zum einmaligen Auspressen von Injektionsflüssigkeit aus einem Behälter. Nach dem einmaligen Auspressen von Injektionsflüssigkeit wird das Injektionsgerät entsorgt. Solche für eine einzige Injektion vorgesehenen Injektionsgeräte sind insbesondere für die Injektion von Medikamenten vorgesehen, die nur in großen Zeitabständen injiziert werden müssen und die Haltbarkeit der Injektionsflüssigkeit kürzer als der Zeitabstand zwischen zwei Injektionen ist.

Vorteilhaft verstellt die Einstellvorrichtung die Lage des Endanschlags in Richtung einer Längsmittelachse des Injektionsgeräts. Dies ermöglicht einen einfachen Aufbau. Die Einstellvorrichtung umfasst vorteilhaft ein im Gehäuse angeordnetes Anschlagelement und einen mindestens teilweise auf die Gehäuseaußenseite ragenden, vom Bediener zu betätigenden Einstellring. Am Einstellring kann der Bediener die Lage des Anschlagelements einstellen. Der Einstellring ist dabei vorteilhaft drehbar gelagert und wird zur Einstellung der auszupressenden Menge an Injektionsflüssigkeit vom Bediener um die Längsmittelachse des Injektionsgeräts gedreht. Ein einfacher Aufbau wird erreicht, wenn der Einstellring über eine Gewindeverbindung mit dem Gehäuse verbunden ist und eine Drehung des Einstellrings eine Bewegung des Einstellrings in Richtung der Längsmittelachse des Injektionsgeräts bewirkt. Durch entsprechende Auslegung der Gewindeverbindung kann eine sehr genaue Einstellung der auszupressenden Menge an Injektionsflüssigkeit erreicht werden.

Vorteilhaft umfasst das Injektionsgerät eine Koppeleinrichtung, die die axiale Lage des Anschlagelements an die axiale Lage des Einstellrings koppelt. Die Koppeleinrichtung umfasst vorteilhaft mindestens ein Koppelelement, das durch eine Öffnung im Gehäuse in eine Führung der Koppeleinrichtung ragt. Vorteilhaft ist das Koppelelement am Anschlagelement festgelegt, und die Führung ist am Einstellring angeordnet. Das Anschlagelement ist im Gehäuse vorteilhaft drehfest geführt. Die Führung wird insbesondere durch eine umlaufende Nut des Einstellrings gebildet. Dadurch, dass das Anschlagelement drehfest im Gehäuse geführt ist, kann die Öffnung im Gehäuse, durch die das Koppelelement ragt, in Umfangsrichtung vergleichsweise schmal ausgebildet werden, da sich das Koppelelement beim Einstellen der auszupressenden Menge an Injektionsflüssigkeit nicht mitdreht. Dadurch wird eine geringe Schwächung des Gehäuses im Bereich des Koppelelements und dadurch eine hohe Stabilität des Injektionsgeräts erreicht. Gleichzeitig ergibt sich ein einfacher Aufbau. Vorteilhaft bewirkt das Koppelelement mit der Öffnung die drehfeste Führung des Anschlagelements. Zusätzliche Mittel zur drehfesten Führung des Anschlagelements werden dadurch nicht benötigt.

Das Injektionsgerät ist vorteilhaft ein Injektionsgerät, bei dem der Dosierkolben einen Rand besitzt, der mit dem Endanschlag zusammenwirkt. Ein derartiges Injektionsgerät kann beispielsweise ein Autoinjektor sein, der zum Auspressen von Injektionsflüssigkeit aus einer Spritze dient. Die Injektionsfeder stützt sich dabei vorteilhaft an der distalen Seite des Rands ab. Dadurch wird ein einfacher Aufbau des Injektionsgeräts mit einer geringen Anzahl von Bauteilen erreicht. Die Injektionsfeder kann im Gehäuse bereits vorgespannt gehalten sein.

Das Injektionsgerät besitzt eine drehfest im Gehäuse gelagerte Injektionshülse, die über eine erste Gewindeverbindung mit einem drehbar und axial unverschiebbar im Gehäuse gelagerten Dosierorgan verbunden ist. Das Dosierorgan ist über eine zweite Gewindeverbindung mit dem Dosierkolben verbunden. Die Injektionshülse besitzt vorteilhaft eine Anschlagfläche, die mit dem Endanschlag zusammenwirkt. Der Endanschlag wirkt demnach nicht direkt mit dem Dosierkolben zusammen, sondern legt die Endlage des Dosierkolbens indirekt über die Injektionshülse, das Dosierorgan und die beiden Gewindeverbindungen fest. Vorteilhaft stützt sich die Injektionsfeder mit ihrem proximalen Ende an der Injektionshülse ab. Bei einem Injektionsgerät, das eine Injektionshülse und ein Dosierorgan besitzt, ist auf dem Dosierorgan vorteilhaft eine für den Bediener sichtbare Skala angebracht, mit der vorteilhaft die Menge der bereits ausgepressten Injektionsflüssigkeit angezeigt wird. Dies ist insbesondere bei Injektionsgeräten vorteilhaft, bei denen der Bediener die Injektion unterbrechen und, beispielsweise nach einer für den Bediener angenehmen Pause, fortsetzen kann.

Vorteilhaft ist am Einstellring eine Skala vorgesehen, aus der die Lage des Endanschlags und damit die eingestellte Menge an auszupressender Injektionsflüssigkeit hervorgeht.

Ein Ausführungsbeispiel der Erfindung wird im Folgenden anhand der Zeichnung erläutert. Es zeigen:
- Fig. 1: eine Seitenansicht eines nicht erfindungsgemäßen Injektionsgeräts in einer ersten Stellung, in der eine erste Menge von auszupressender Injektionsflüssigkeit eingestellt ist,
- Fig. 2: einen Schnitt entlang der Linie II-II in Fig. 1,
- Fig. 3: eine Seitenansicht auf das Injektionsgerät in Richtung des Pfeils III in Fig. 1,
- Fig. 4: eine vergrößerte Schnittdarstellung entlang der Linie IV-IV in Fig. 3,
- Fig. 5: eine Seitenansicht des Injektionsgeräts nach dem Abnehmen der Sicherheitskappe,
- Fig. 6: einen Schnitt entlang der Linie VI-VI in Fig. 5,
- Fig. 7: eine Seitenansicht des Injektionsgeräts nach dem Verschieben des Bedienelements in proximale Richtung,
- Fig. 8: einen Schnitt entlang der Linie VIII-VIII in Fig. 7,
- Fig. 9: eine Seitenansicht des Injektionsgeräts nach dem Auspressen der Injektionsflüssigkeit,
- Fig. 10: einen Schnitt entlang der Linie X-X in Fig. 9,
- Fig. 11: eine Seitenansicht in Richtung des Pfeils XI-XI,
- Fig. 12: eine ausschnittsweise Schnittdarstellung entlang der Linie XII-XII in Fig. 11,
- Fig. 13: eine Seitenansicht des Injektionsgeräts vor dem Injektionsvorgang mit einer zweiten, maximalen eingestellten Menge an Injektionsflüssigkeit,
- Fig. 14: einen Schnitt entlang der Linie XIV-XIV in Fig. 13,
- Fig. 15: eine ausschnittsweise Seitenansicht in Richtung des Pfeils XV in Fig. 13,
- Fig. 16: einen Schnitt entlang der Linie XVI-XVI in Fig. 15,
- Fig. 17: eine perspektivische Darstellung eines unteren Gehäuseteils des Injektionsgeräts,
- Fig. 18: eine Seitenansicht des unteren Gehäuseteils,
- Fig. 19: eine Seitenansicht in Richtung des Pfeils XIX in Fig. 18,
- Fig. 20: eine Seitenansicht in Richtung des Pfeils XX in Fig. 19,
- Fig. 21: einen Schnitt entlang der Linie XXI-XXI in Fig. 20,
- Fig. 22: eine perspektivische Darstellung eines oberen Gehäuseteils des Injektionsgeräts,
- Fig. 23: eine Seitenansicht des oberen Gehäuseteils,
- Fig. 24: eine Seitenansicht in Richtung des Pfeils XXIV in Fig. 23,
- Fig. 25: einen Schnitt entlang der Linie XXV-XXV in Fig. 24,
- Fig. 26: eine perspektivische Darstellung eines Anschlagelements des Injektionsgeräts,
- Fig. 27: eine Seitenansicht des Anschlagelements,
- Fig. 28: eine Seitenansicht in Richtung des Pfeils XXVIII in Fig. 27,
- Fig. 29: eine perspektivische Darstellung eines Einstellrings des Injektionsgeräts,
- Fig. 30: eine Seitenansicht des Einstellrings,
- Fig. 31: einen Schnitt entlang der Linie XXXI-XXXI in Fig. 30,
- Fig. 32: eine perspektivische Darstellung eines Bedienelements des Injektionsgeräts,
- Fig. 33: eine Seitenansicht des Bedienelements,
- Fig. 34: einen Schnitt entlang der Linie XXXIV-XXXIV in Fig. 33,
- Fig. 35: eine Seitenansicht eines Dosierkolbens des Injektionsgeräts,
- Fig. 36: einen Schnitt entlang der Linie XXXVI-XXXVI in Fig. 35,
- Fig. 37: eine Seitenansicht eines erfindungsgemäßen Injektionsgeräts,
- Fig. 38: eine Seitenansicht in Richtung des Pfeils XXXVIII in Fig. 37,
- Fig. 39: einen Schnitt entlang der Linie XXXIX-XXXIX in Fig. 38,
- Fig. 40: eine Seitenansicht des Injektionsgeräts nach dem Verschieben des Bedienelements in proximale Richtung,
- Fig. 41: eine Seitenansicht in Richtung des Pfeils XLI in Fig. 40,
- Fig. 42: einen Schnitt entlang der Linie XLII-XLII in Fig. 41,
- Fig. 43: eine Seitenansicht des Injektionsgeräts nach dem Injektionsvorgang,
- Fig. 44: eine Seitenansicht in Richtung des Pfeils XLIV in Fig. 43,
- Fig. 45: einen Schnitt entlang der Linie XLV-XLV in Fig. 44,
- Fig. 46: eine Seitenansicht des Injektionsgeräts vor dem Injektionsvorgang mit einer zweiten eingestellten auszupressenden Menge an Injektionsflüssigkeit,
- Fig. 47: eine Seitenansicht in Richtung des Pfeils XLVII in Fig. 46,
- Fig. 48: einen Schnitt entlang der Linie XLVIII-XLVIII in Fig. 47,
- Fig. 49: eine perspektivische Darstellung eines oberen Gehäuseteils des Injektionsgeräts,
- Fig. 50: eine Seitenansicht des oberen Gehäuseteils,
- Fig. 51: einen Schnitt entlang der Linie LI-LI in Fig. 50,
- Fig. 52: einen Schnitt entlang der Linie LII-LII in Fig. 51,
- Fig. 53: einen Schnitt entlang der Linie LIII-LIII in Fig. 51,
- Fig. 54: eine Seitenansicht einer Injektionshülse des Injektionsgeräts,
- Fig. 55: eine Seitenansicht in Richtung des Pfeils LV in Fig. 54,
- Fig. 56: einen Schnitt entlang der Linie LVI-LVI in Fig. 55,
- Fig. 57: eine Seitenansicht in Richtung des Pfeils LVII in Fig. 54,
- Fig. 58: eine Seitenansicht in Richtung des Pfeils LVIII in Fig. 54,
- Fig. 59: eine perspektivische Darstellung eines Anschlagelements des Injektionsgeräts,
- Fig. 60: eine Seitenansicht des Anschlagelements,
- Fig. 61: eine Draufsicht in Richtung des Pfeils 61 in Fig. 60,
- Fig. 62: einen Schnitt entlang der Linie LXII-LXII in Fig. 60,
- Fig. 63: eine Seitenansicht eines Einstellrings des Injektionsgeräts,
- Fig. 64: einen Schnitt entlang der Linie LXIV-LXIV in Fig. 63,
- Fig. 65 und Fig. 66: perspektivische Darstellungen eines Bedienelements des Injektionsgeräts,
- Fig. 67: eine Seitenansicht des Bedienelements,
- Fig. 68: eine Ansicht des Bedienelements in Richtung des Pfeils LXVIII in Fig. 67,
- Fig. 69: einen Schnitt entlang der Linie LXIX-LXIX in Fig. 67,
- Fig. 70: eine Seitenansicht eines Mitnehmers des Injektionsgeräts,
- Fig. 71: einen Schnitt entlang der Linie LXXI-LXXI in Fig. 70,
- Fig. 72: eine Ansicht des Mitnehmers in Richtung des Pfeils LXXII in Fig. 70,
- Fig. 73: eine Ansicht des Mitnehmers in Richtung des Pfeils LXXIII in Fig. 70,
- Fig. 74: eine Seitenansicht eines Dosierorgans des Injektionsgeräts,
- Fig. 75: einen Schnitt entlang der Linie LXXV-LXXV in Fig. 74,
- Fig. 76: eine Ansicht des Dosierorgans in Richtung des Pfeils LXXVI in Fig. 75,
- Fig. 77: eine perspektivische Darstellung eines Dosierkolbens des Injektionsgeräts,
- Fig. 78: eine Seitenansicht des Dosierkolbens,
- Fig. 79: eine Seitenansicht des Dosierkolben in Richtung des Pfeils LXXIX in Fig. 78,
- Fig. 80: einen Schnitt entlang der Linie LXXX-LXXX in Fig. 79.

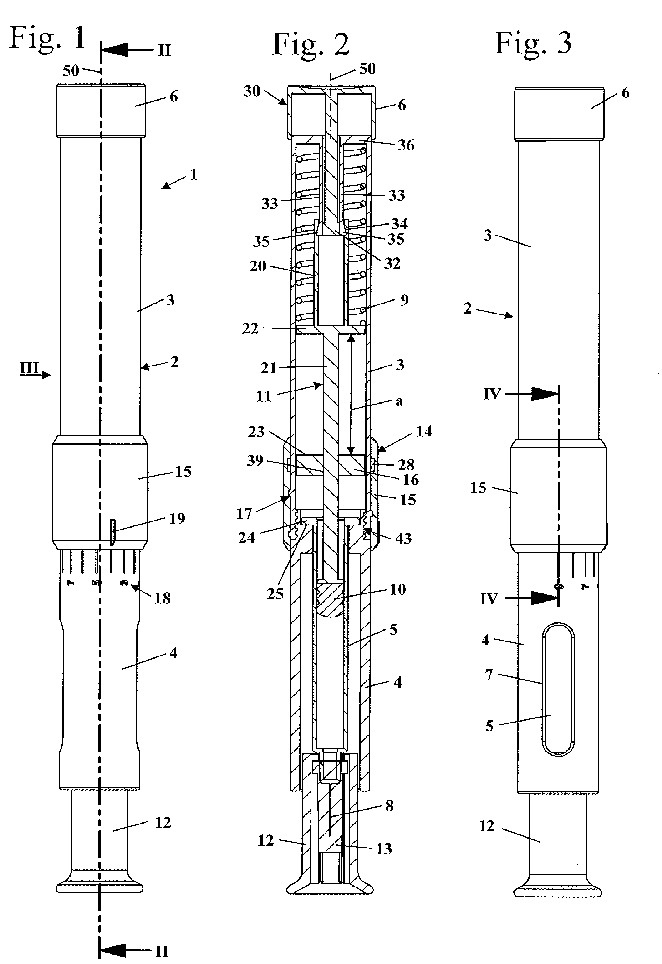

Fig. 1 zeigt ein nicht erfindungsgemäßes Injektionsgerät 1, das zum einmaligen, automatischen Auspressen einer Dosis von Injektionsflüssigkeit aus einem Behälter, beispielsweise einer Spritze, vorgesehen ist. Derartige Injektionsgeräte werden auch als Autoinjektoren bezeichnet. Das Injektionsgerät 1 besitzt ein Gehäuse 2, das ein oberes, distales Gehäuseteil 3 und ein unteres, proximales Gehäuseteil 4 umfasst. Am oberen Gehäuseteil 3 ist ein Einstellring 15 drehbar gelagert. Der Einstellring 15 dient zur Einstellung der auszupressenden Menge an Injektionsflüssigkeit. Dies ist insbesondere für Injektionsflüssigkeiten vorteilhaft, bei denen die benötigte Menge beispielsweise vom Körpergewicht des Anwenders abhängt. Dadurch, dass der Bediener in konstruktiv vorgegebenen Grenzen die auszupressende Menge an Injektionsflüssigkeit einstellen kann, muss nicht für jede mögliche Menge an Injektionsflüssigkeit ein separates Injektionsgerät vorgehalten werden. Der Einstellring 15 trägt eine Markierung 19, die an einer am unteren Gehäuseteil 4 aufgebrachten Skala 18 die eingestellte Menge an auszupressender Injektionsflüssigkeit anzeigt. Am distalen Ende des oberen Gehäuseteils 3 ist ein Bedienelement 6 angeordnet. Am proximalen Ende ist eine Sicherheitskappe 12 angeordnet, die eine Injektionsnadel 8 (Fig. 2) des Injektionsgeräts 1 abdeckt.

Das distale Ende des Injektionsgeräts 1 ist das der Injektionsnadel 8 abgewandt liegende Ende. Mit "proximal" wird die Seite des Injektionsgeräts 1 bezeichnet, die bei einer Injektion der Einstichstelle zugewandt liegt, und mit "distal" die Seite, die der Einstichstelle abgewandt liegt. Die proximale Richtung bezeichnet die Injektionsrichtung, also die Richtung zur Injektionsnadel hin bzw. die Richtung, in der die Injektionsflüssigkeit aus dem Behälter ausgepresst wird. Die distale Richtung bezeichnet die entgegengesetzte Richtung, also von der Injektionsnadel 8 weg.

Fig. 2 zeigt den Aufbau des Injektionsgeräts 1 im Einzelnen. Im unteren Gehäuseteil 4 ist ein Behälter 5 mit Injektionsflüssigkeit angeordnet. Beispielsweise ist der Behälter 5 eine Spritze, die sich mit ihrem distalen Rand 24 an einem Absatz 25 des unteren Gehäuseteils 4 abstützt. Die Injektionsnadel 8 ist von einem Nadelschutz 13 umgeben, der mit der Sicherheitskappe 12 fest verbunden ist und der beim Abziehen der Sicherheitskappe 12 ebenfalls abgezogen wird. Im Behälter 5 ist ein Stopfen 10 angeordnet, an dem ein Dosierkolben 11 des Injektionsgeräts 1 anliegt. Der Dosierkolben 11 bewegt sich beim Auspressen von Injektionsflüssigkeit in proximale Richtung und verschiebt dadurch den Stopfen 10 in proximale Richtung, so dass Injektionsflüssigkeit ausgepresst wird. Der Dosierkolben 11 besitzt eine Kolbenstange 21, die in proximale Richtung ragt, sowie einen Hülsenabschnitt 20, der eine etwa zylindrische Form hat und in distale Richtung ragt. Zwischen dem Hülsenabschnitt 20 und der Kolbenstange 21 ist ein quer zur Längsmittelachse 50 des Injektionsgeräts 1 verlaufender Rand 22 ausgebildet, der beispielsweise die Form einer Scheibe hat. An seinem distalen Ende besitzt der Hülsenabschnitt 20 eine Vertiefung 34, in die Verriegelungselemente 35 des Bedienelements 6 ragen. Die Verriegelungselemente 35 sind beispielhaft an Haltearmen 33 des oberen Gehäuseteils 3 ausgebildet. Die Haltearme 33 erstrecken sich parallel zu einer Längsmittelachse 50 des Injektionsgeräts 1. Radial innerhalb der Verriegelungselemente 35 ist ein Sperrabschnitt 32 des Bedienelements 6 angeordnet. Der Sperrabschnitt 32 ist so ausgebildet, dass die Verriegelungselemente 35 sich nicht aus der Vertiefung 34 radial nach innen bewegen können. Dadurch fixieren die Verriegelungselemente 35 den Dosierkolben 11 in der in Fig. 2 gezeigten Position, so dass sich der Dosierkolben 11 nicht in proximale Richtung bewegen kann.

Im oberen Gehäuseteil 3 ist eine Injektionsfeder 9 angeordnet, die sich mit ihrem distalen Ende an einer distalen Wand 36 des oberen Gehäuseteils 3 abstützt und mit ihrem proximalen Ende am Rand 22 des Dosierkolbens 11. Die Injektionsfeder 9 ist vorgespannt. Sobald die Verriegelungselemente 35 den Dosierkolben 11 freigeben, drückt die Injektionsfeder 9 den Dosierkolben 11 in proximale Richtung, so dass automatisch Injektionsflüssigkeit ausgepresst wird.

Zur Einstellung der auszupressenden Menge an Injektionsflüssigkeit besitzt das Injektionsgerät 1 eine Einstellvorrichtung 14. Die Einstellvorrichtung 14 umfasst den Einstellring 15. In der in den Fig. 1 und 2 gezeigten Stellung weist die Markierung 19 auf den Wert 4 der Skala 18. Dies bedeutet, dass zusätzlich zu einer Grunddosis vier Einheiten von Injektionsflüssigkeit ausgepresst werden. Die Einstellvorrichtung 14 umfasst ein innerhalb des Gehäuses 2 angeordnetes Anschlagelement 16, das beispielhaft als Scheibe ausgebildet ist. Das Anschlagelement 16 besitzt eine mittige Öffnung 39, durch die die Kolbenstange 21 ragt. An der distalen Seite des Anschlagelements 16 ist ein Endanschlag 23 für den Rand 22 gebildet. Im Beispiel wird der Endanschlag 23 durch die gesamte distale Stirnseite des Anschlagelements 16 gebildet. Wie Fig. 2 auch zeigt, ist der Einstellring 15 über eine Gewindeverbindung 17 am Gehäuse 2 gehalten. An seiner Innenseite besitzt der Einstellring 15 auf der Höhe des Anschlagelements 16 eine Führung 28, die als Nut ausgebildet ist und deren Funktion im Folgenden noch näher erläutert wird. Wie Fig. 2 zeigt, besitzt der Rand 22 zum Endanschlag 23 einen in axialer Richtung gemessenen Abstand a.

Wie Fig. 2 zeigt, sind das obere Gehäuseteil 3 und das untere Gehäuseteil 4 beispielsweise über eine Gewindeverbindung 43 miteinander verbunden. Auch eine andere Verbindung der Gehäuseteile 3 und 4 kann vorteilhaft sein. Das Injektionsgerät 1 besitzt zwei Sichtfenster 7 im unteren Gehäuseteil 4, durch die der Behälter 5 sichtbar ist. In Fig. 3 ist eines der Sichtfenster 7 gezeigt.

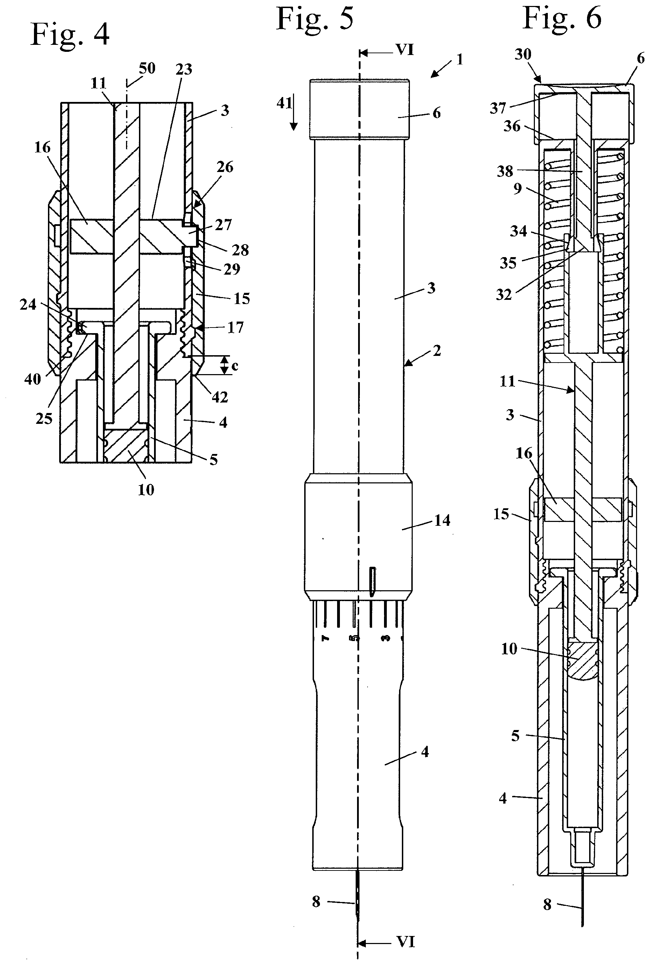

Wie Fig. 4 zeigt, besitzt der Einstellring 15 eine proximale Stirnseite 42. Das obere Gehäuseteil besitzt eine proximale Stirnseite 40. Die Stirnseite 40 und die Stirnseite 42 besitzen bei der eingestellten Menge an Injektionsflüssigkeit einen in Richtung der Längsmittelachse gemessenen Abstand c zueinander. Fig. 4 zeigt auch eine Koppeleinrichtung 26 der Einstellvorrichtung 14 im Einzelnen. Die Koppeleinrichtung 26 koppelt die axiale Stellung des Anschlagelements 16 an die axiale Stellung des Einstellrings 15. Die Koppeleinrichtung 26 umfasst einen Zapfen 27 des Anschlagelements 16, der durch eine Öffnung 29 des oberen Gehäuseteils 3 in die Führung 28 des Einstellrings 25 ragt. Beispielhaft entspricht die Erstreckung des Zapfens 27 in Richtung der Längsmittelachse 50 dabei der Höhe der Nut 28, so dass der Zapfen 27 weitgehend spielfrei in der Führung 28 gehalten ist. Dreht der Bediener den Einstellring 15, so bewegt sich der Einstellring 15 aufgrund der Gewindeverbindung 17 in Richtung der Längsmittelachse 50. Je nach Drehrichtung bewegt sich der Einstellring 15 in distale Richtung oder in proximale Richtung. Über die Führung 28, in die der Zapfen 27 eingreift, nimmt der Einstellring 15 bei seiner axialen Bewegung das Anschlagelement 16 mit. Dadurch wird die Lage des Endanschlags 23 im Gehäuse 2 in Richtung der Längsmittelachse 50 verstellt. Aufgrund der vergleichsweise geringen Steigung der Gewindeverbindung 17 ist eine feinfühlige Einstellung der axialen Lage des Anschlagelements 16 und damit der auszupressenden Dosis möglich.

Zum Durchführen einer Injektion nimmt der Bediener nach dem Einstellen der auszupressenden Menge an Injektionsflüssigkeit die Sicherheitskappe 12 ab. Die Fig. 5 und 6 zeigen das Injektionsgerät 1 mit abgenommener Sicherheitskappe. Das Bedienelement 6 ist topfförmig mit einem ins Innere des oberen Gehäuseteils 3 ragenden, stegförmigen Verbindungsabschnitt 38 ausgebildet, der den Sperrabschnitt 32 trägt. Die Innenseite 37 des Bedienelements 6 bildet mit der distalen Wand 36 des oberen Gehäuseteils 3 einen Anschlag für die proximale Position 31 des Bedienelements 6.

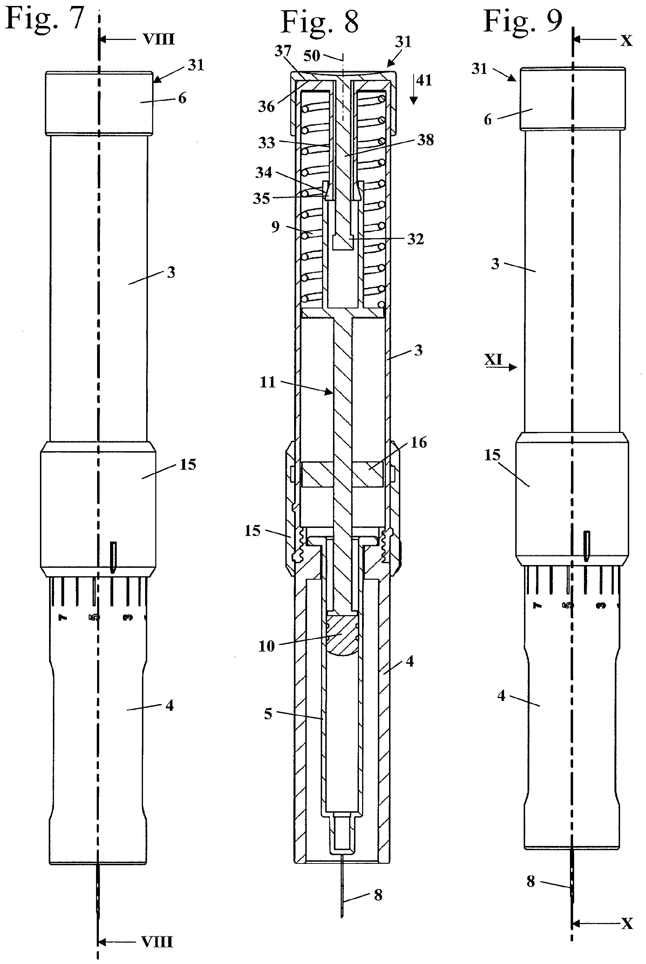

In den Figuren 1 bis 6 befindet sich das Bedienelement 6 in seiner distalen Position 30. Zum Auslösen einer Injektion ist das Bedienelement 6 in proximale Richtung 41 zu bewegen. Diese Position ist in den Fig. 7 und 8 gezeigt. Das Bedienelement 6 befindet sich in seiner proximalen Position 31. Die Innenseite 37 des Bedienelements 6 liegt an der distalen Wand 36 des oberen Gehäuseteils 3 an. Der Sperrabschnitt 32 hat sich in proximale Richtung 41 bewegt. Der Verbindungabschnitt 38, der durch die Bewegung i proximale Richtung 41 in den Bereich der Verrieglungselemente 35 gekommen ist, ist schmaler ausgebildet als der Sperrabschnitt 32. Dadurch können die Verriegelungselemente 35 in der proximalen Position 31 des Bedienelements 6 radial nach innen verschwenken. Die Vertiefung 34 und die Verriegelungselemente 35 sind abgeschrägt ausgebildet. Die Injektionsfeder 9 übt auf den Dosierkolben 11 eine Kraft in proximale Richtung 41 aus. Diese Kraft bewirkt eine Bewegung des Dosierkolbens 11 in proximale Richtung 41, wobei die Verriegelungselemente 35 über die Abschrägungen radial nach innen ausgelenkt werden. Der Dosierkolbenl 1 kann sich dadurch frei in proximale Richtung 41 bewegen und presst dabei Injektionsflüssigkeit aus dem Behälter 5 aus.

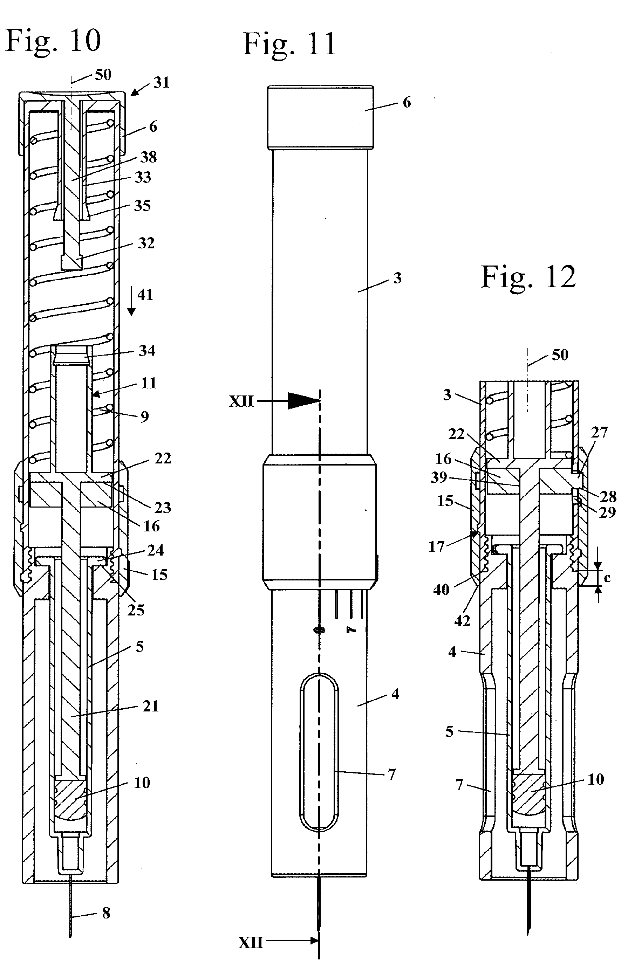

Die Fig. 9 und 10 zeigen das Injektionsgerät 1 nach dem Injektionsvorgang. Das Bedienelement 6 befindet sich in seiner proximalen Position 31. Auch der Dosierkolben 11 befindet sich in seiner proximalen Position. Der Rand 22 liegt am Endanschlag 23 des Anschlagelements 16 an. Der Stopfen 10 wurde nicht vollständig bis an das proximale Ende des Behälters 5 gedrückt, so dass noch eine Restmenge von Injektionsflüssigkeit im Behälter 5 angeordnet ist. Wie die Fig. 11 und 12 zeigen, hat sich die Lage des Anschlagelements 16 im Gehäuse 2 nicht geändert. Die Stirnseiten 40 und 42 besitzen weiterhin den Abstand c zueinander. Der Dosierkolben 11 stützt sich über den Rand 22 und den Zapfen 27 des Anschlagelements 16 in der Führung 28 des Einstellrings 15 ab. Die Steigung der Gewindeverbindung 17 ist so gering, dass der Zapfen 27 keine Verschiebung des Einstellrings 15 in proximale Richtung bewirken kann.

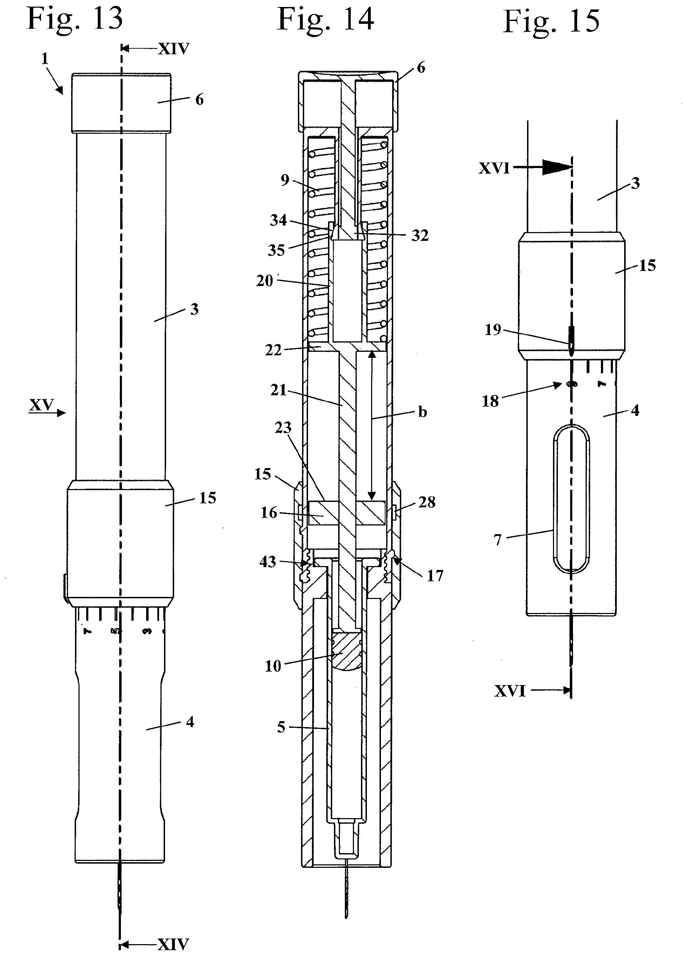

Die Fig. 13 bis 16 zeigen das Injektionsgerät mit einer zweiten eingestellten Menge auszupressender Injektionsflüssigkeit. Die zweite Menge an auszupressender Injektionsflüssigkeit entspricht neun zusätzlichen Einheiten von Injektionsflüssigkeit, also der maximal einstellbaren Dosis des Injektionsgeräts 1.

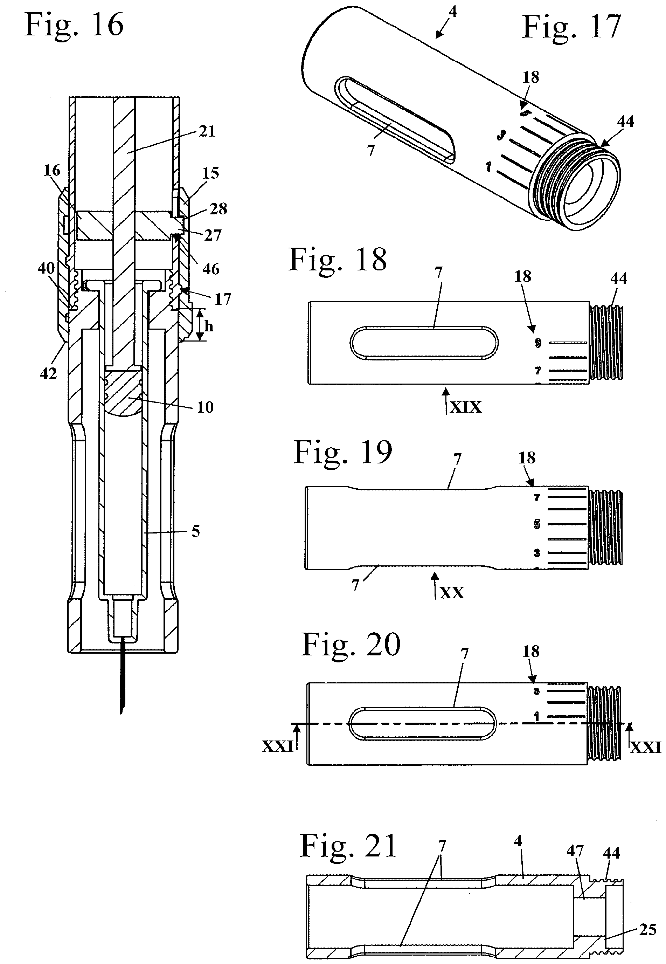

Wie Fig. 14 zeigt, besitzt der Rand 22 zum Endanschlag 23 bei der eingestellten Dosis von neun zusätzlichen Einheiten einen Abstand b. Der Abstand b ist dabei größer als der Abstand a (Fig. 2) bei der ersten eingestellten Dosis von vier zusätzlichen Einheiten. Gegenüber der in Fig. 2 gezeigten Position des Anschlagelements 16 hat sich das Anschlagelement 16 in proximale Richtung bewegt. Wie Fig. 16 zeigt, besitzt die Stirnseite 40 des oberen Gehäuseteils 3 zur proximalen Stirnseite 42 des Einstellrings 15 in dieser Position einen Abstand h, der größer als der Abstand c bei der ersten eingestellten Dosis (Fig. 12) ist. Dadurch kann sich der Dosierkolben 11 bei der zweiten eingestellten Dosis weiter in distale Richtung bewegen und dadurch eine größere Menge von Injektionsflüssigkeit aus dem Behälter 5 auspressen. Das proximale Ende der Öffnung 29 bildet einen ersten, proximalen Anschlag 46 für den Zapfen 27. Wie Fig. 16 zeigt, liegt der Zapfen 27 bei der maximalen eingestellten Menge an Injektionsflüssigkeit am Anschlag 46 an.

Die Fig. 17 bis 21 zeigen den Aufbau des unteren Gehäuseteils 4 im Einzelnen. Wie die Figuren zeigen, besitzt das obere Gehäuseteil 4 an seiner distalen, in den Fig. 18 bis 21 rechts gezeigten Seite ein Außengewinde 44, das Teil der Gewindeverbindung 43 zum oberen Gehäuseteil 3 ist. In den Fig. 18 bis 20 ist auch die Skala 18 im Einzelnen gezeigt. Die Skala 18 reicht beispielsweise von 1 bis 9, so dass mit der Einstellvorrichtung 14 eine bis neun zusätzliche Einheiten von Injektionsflüssigkeit injiziert werden können. Wie groß eine Einheit ist, ist abhängig von der Steigung der Gewindeverbindung 17. Wie Fig. 21 zeigt, besitzt das obere Gehäuseteil 21 benachbart zum Absatz 25 eine Aufnahmeöffnung 47 für den Behälter 5.

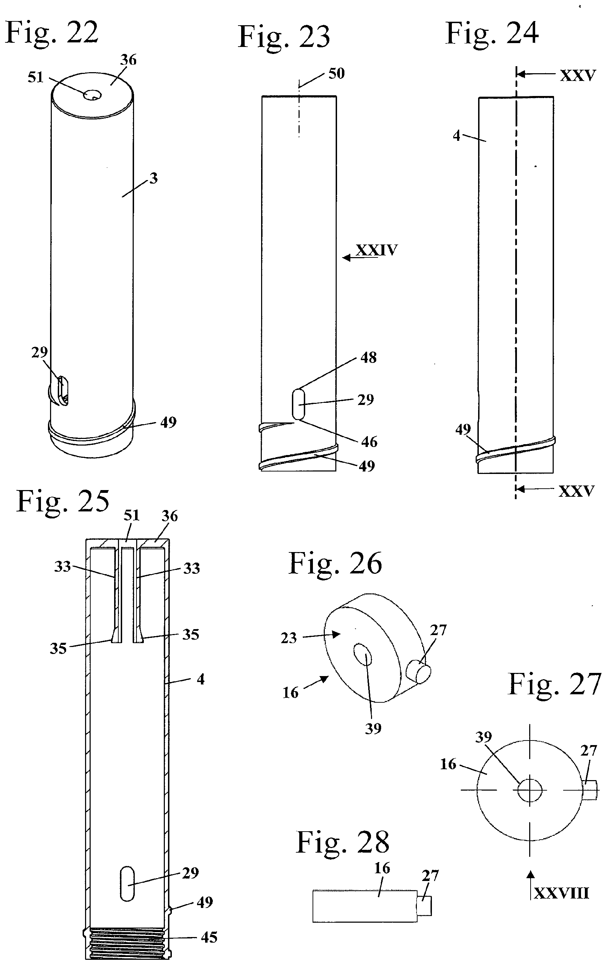

Die Fig. 22 bis 25 zeigen das obere Gehäuseteil 3 im Einzelnen. Die distale Wand 36 besitzt eine Durchtrittsöffnung 51 für den Verbindungsabschnitt 38 des Bedienelements 6. In den Fig. 22 und 23 ist auch die Öffnung 29 sichtbar. Die Öffnung 29 ist als Langloch ausgebildet, das sich parallel zur Längsmittelachse 50 erstreckt. Das proximale Ende der Öffnung 29 bildet den ersten, proximalen Anschlag 46 für den Zapfen 27. Der Anschlag 46 begrenzt die maximal auszupressende Menge an Injektionsflüssigkeit. Das distale Ende der Öffnung 29 bildet einen zweiten, distalen Anschlag 48 für den Zapfen 27, der die minimal einstellbare Menge an auszupressender Injektionsflüssigkeit festlegt. Wie die Fig. 22 bis 25 zeigen, trägt das obere Gehäuseteil 3 an seinem distalen Ende ein Außengewinde 49, das Teil der Gewindeverbindung 17 ist. Wie Fig. 25 zeigt, ist am distalen Ende auch ein Innengewinde 45 vorgesehen, das mit dem Außengewinde 44 des unteren Gehäuseteils 4 die Gewindeverbindung 43 zwischen den beiden Gehäuseteilen 3 und 4 bildet. In Fig. 25 ist auch die Gestaltung der beiden Haltearme 33 gezeigt, die sich von der Innenseite der Wand 36 in proximale Richtung erstrecken. Die Haltearme 33 sind am Umfang der Durchtrittsöffnung 52 angeordnet.

Die Fig. 26 bis 28 zeigen die Gestaltung des Anschlagelements 16 im Einzelnen. Das Anschlagelement 16 ist als Scheibe ausgebildet, dessen distale Seite den Endanschlag 23 bildet. Der Zapfen 27 ist beispielhaft zylindrisch ausgebildet und ragt radial nach außen. Auch eine andere Gestaltung des Koppelelements, insbesondere eine eckige Gestaltung, kann jedoch vorteilhaft sein.

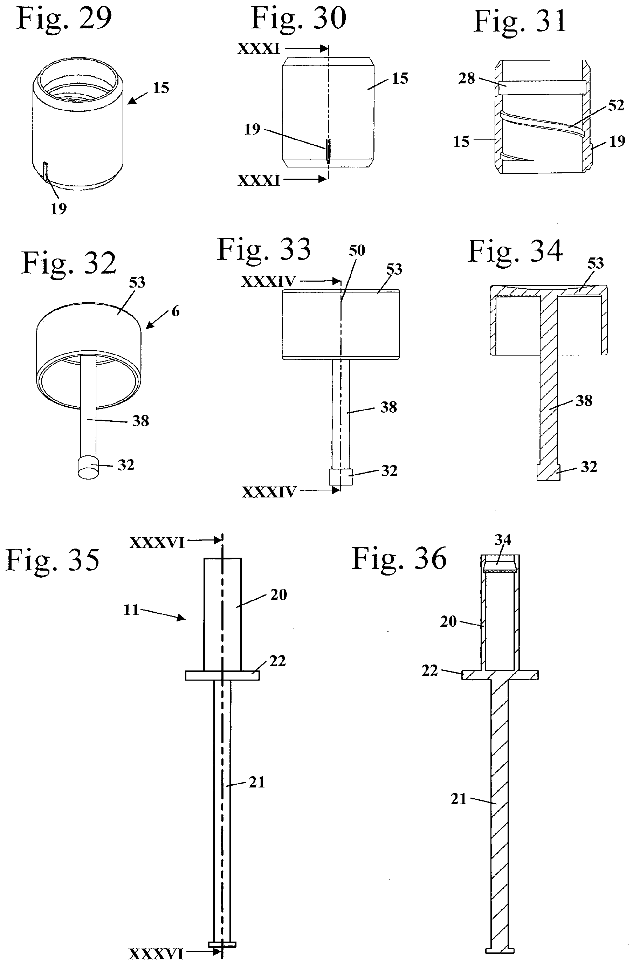

Die Fig. 29 bis 31 zeigen die Gestaltung des Einstellrings 15. Der Einstellring 15 ist hülsenförmig ausgebildet und vollständig an der Außenseite des Gehäuses 2 angeordnet. Es kann jedoch auch vorgesehen sein, dass der Einstellring 15 sich durch die Öffnung 29 zum Anschlagelement 16 erstreckt. Allerdings muss die Öffnung 29 dann eine entsprechend große Erstreckung in Umfangsrichtung aufweisen, was zu einer Verringerung der Stabilität des Gehäuses 2 führen kann. Der Einstellring 15 besitzt ein Innengewinde 52, das mit dem Außengewinde 49 des oberen Gehäuseteils 3 die Gewindeverbindung 17 bildet. In seinem distalen Bereich besitzt der Einstellring 15 an seinem Innenumfang die Führung 28, die als umlaufende Nut ausgebildet ist. Die Höhe der Führung 28 entspricht der Höhe des Zapfens 27, so dass eine spielfreie Einstellung der auszupressenden Menge an Injektionsflüssigkeit möglich ist. Es kann jedoch auch Spiel zwischen der Führung 28 und dem Zapfen 27 vorgesehen sein, da die Position des Zapfens 27 in der Führung 28 am Ende der Injektion aufgrund der Kraft der Injektionsfeder 9 vorgegeben ist.

Wie die Fig. 32 bis 34 zeigen, besitzt das Bedienelement 6 an seiner distalen Seite einen Betätigungsabschnitt 53, der sich etwa senkrecht zur Längsmittelachse 50 erstreckt und an dem der Bediener das Bedienelement 6 in proximale Richtung 41 (Fig. 8) drücken kann. Der Verbindungsabschnitt 38 ist stabförmig ausgebildet und ragt von der Mitte des Betätigungsabschnitts 53 in proximale Richtung. Der Verbindungsabschnitt 38 und der Sperrabschnitt 32 sind jeweils zylindrisch ausgebildet, wobei der Außendurchmesser des Sperrabschnitts 32 größer als der des Verbindungsabschnitts 38 ist.

Die Fig. 35 und 36 zeigen den Dosierkolben 11. Der Hülsenabschnitt 20 besitzt an seinem distalen Ende an seiner Innenseite die Vertiefung 34, die beispielhaft als umlaufende Nut ausgebildet ist. Es kann jedoch auch vorgesehen sein, dass für jedes Verriegelungselement 35 eine separate Vertiefung 34 am Dosierkolben 11 ausgebildet ist. Die Gestaltung und Anzahl der Verriegelungselemente 35 kann auf die von der Injektionsfeder 9 ausgeübte Kraft angepasst ausgelegt werden.

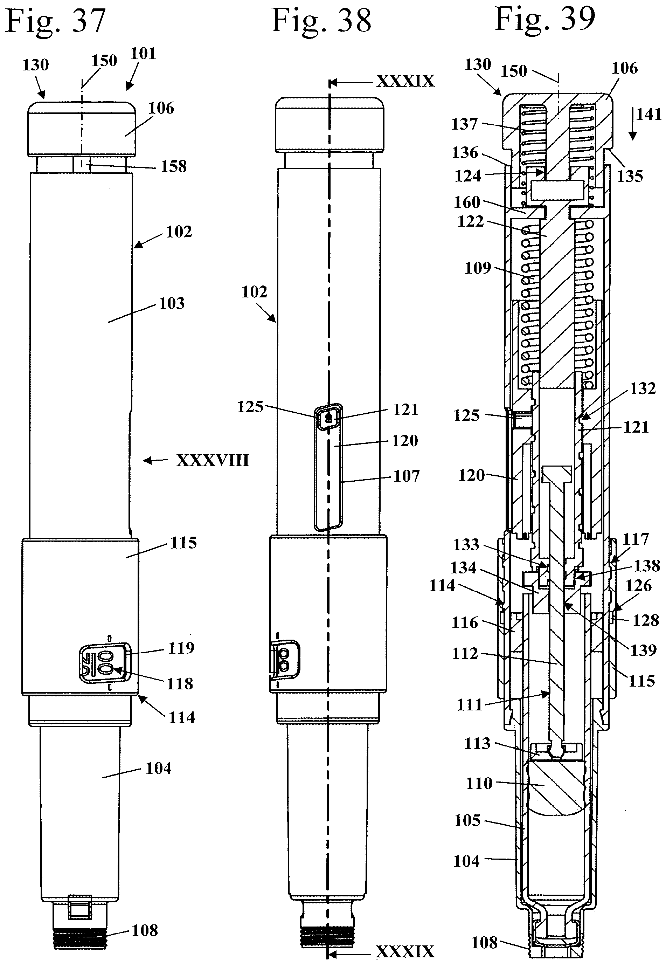

Die Fig. 37 bis 80 zeigen ein erfindungsgemäßes Ausführungsbeispiel eines Injektionsgeräts 101. Das Injektionsgerät 101 besitzt ein Gehäuse 102, das ein oberes, distales Gehäuseteil 103 und ein unteres, proximales Gehäuseteil 104 umfasst. An der distalen Seite des oberen Gehäuseteils 103 ist ein Bedienelement 106 angeordnet, der sich in den Fig. 37 bis 39 in seiner distalen Position 130 befindet. An seinem proximalen Ende besitzt das Injektionsgerät 101 eine Nadelaufnahme 108, an der eine Injektionsnadel fixiert werden kann. Zur Einstellung der auszupressenden Menge an Injektionsflüssigkeit besitzt das Injektionsgerät 101 eine Einstellvorrichtung 114, die einen drehbar an der Außenseite des Gehäuses 102 gelagerten Einstellring 115 umfasst. Der Einstellring 115 besitzt ein Sichtfenster 119, durch das eine Skala 118 sichtbar ist, die an der Außenseite des oberen Gehäuseteils 103 aufgebracht ist.

Wie Fig. 38 zeigt, besitzt das obere Gehäuseteil 103 ein Sichtfenster 107. Durch das Sichtfenster 107 ist die Außenseite einer im Gehäuse 102 angeordneten Injektionshülse 120 sichtbar. Die Injektionshülse 120 besitzt eine Öffnung 125, die auch in Fig. 39 gezeigt ist, und durch die ein innerhalb der Injektionshülse 120 angeordnetes Dosierorgan 121 sichtbar ist, das an seiner Außenseite die Skala 118 trägt.

Wie Fig. 39 zeigt, ist im unteren Gehäuseteil 104 ein Behälter 105, im Ausführungsbeispiel eine Karpule, angeordnet. Vorteilhaft ist auch das Injektionsgerät 101 zum einmaligen Auspressen einer Dosis von Injektionsflüssigkeit vorgesehen. Im Behälter 105 ist ein Stopfen 110 angeordnet, an dem eine Kolbenscheibe 113 eines Dosierkolbens 111 anliegt. Zum Auspressen von Injektionsflüssigkeit wird der Dosierkolben 111 in proximale Richtung 141 bewegt und verschiebt dadurch den Stopfen 110, so dass Injektionsflüssigkeit ausgepresst wird. Der Dosierkolben 111 besitzt eine Kolbenstange 112, die in einer Führung 139 drehfest und axial verschiebbar im Gehäuse 102 gelagert ist. Die Führung 139 ist an einem Lagerabschnitt 134 des Gehäuses 102 ausgebildet. Das Dosierorgan 121 ist über eine Gewindeverbindung 133 mit dem Dosierkolben 111 verbunden. Das Dosierorgan 121 ist am Lagerabschnitt 134 über ein Drehlager 138 drehbar und axial fest gelagert. Das Dosierorgan 121 ist über eine Gewindeverbindung 132 mit der Injektionshülse 120 verbunden. Im Gehäuse 102 ist eine Injektionsfeder 109 angeordnet, die sich mit ihrem distalen Ende an einer Zwischenwand 160 des oberen Gehäuseteils 3 und mit ihrem proximalen Ende an der Injektionshülse 120 abstützt. Die Injektionsfeder 109 ist vorteilhaft bereits vorgespannt, so dass der Bediener die Injektionsfeder 109 nicht spannen muss. Dies ist insbesondere dann vorteilhaft, wenn die Injektionsflüssigkeit hochviskos ist, so dass die Injektionsfeder 109 sehr stark ausgelegt sein muss und zum Spannen der Injektionsfeder 109 vergleichsweise große Kräfte benötigt werden.

Das Injektionsgerät 101 besitzt einen Mitnehmer 122, der über eine Kupplung 124 mit dem Bedienelement 106 verbunden ist. In der in Fig. 38 gezeigten distalen Position 130 des Bedienelements 106 sind das Bedienelement 106 und der Mitnehmer 122 über die Kupplung 124 drehfest miteinander verbunden. Der Mitnehmer 124 ist drehfest mit dem Dosierorgan 121 verbunden. Das Bedienelement 106 ist über Führungsnuten 158 (Fig. 37) drehfest im Gehäuse 102 geführt. In der in den Fig. 37 bis 39 gezeigten distalen Position 130 des Bedienelements 106 können sich demnach der Mitnehmer 122, das Dosierorgan 121, die Injektionshülse 120 und der Dosierkolben 111 nicht um eine Längsmittelachse 150 des Injektionsgeräts 101 drehen.

Die Einstellvorrichtung 114 umfasst ein Anschlagelement 116, das im Gehäuse 102 angeordnet ist. Im Ausführungsbeispiel ist das Anschlagelement 116 hülsenförmig ausgebildet und erstreckt sich am Außenumfang des Behälters 105. Das Anschlagelement 116 ist über eine Koppeleinrichtung 126 an die axiale Position des Einstellrings 115 gekoppelt. Der Einstellring 115 ist über eine Gewindeverbindung 117 mit dem oberen Gehäuseteil 103 verbunden. Wird der Einstellring 115 zur Einstellung der auszupressenden Menge an Injektionsflüssigkeit vom Bediener um die Längsmittelachse 150 gedreht, so bewegt sich der Einstellring 115 aufgrund der Gewindeverbindung 117 in Richtung der Längsmittelachse 150. Über die Koppeleinrichtung 126 wird das Anschlagelement 116 mitgenommen und entsprechend in axialer Richtung bewegt.

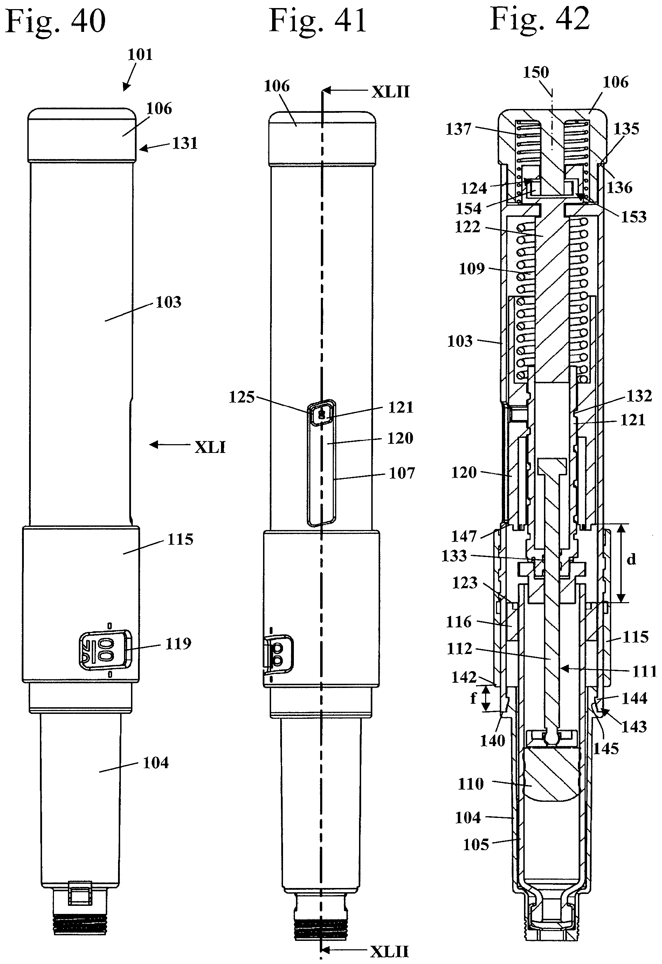

Das Bedienelement 106 ist von einer Betätigungsfeder 137 in Richtung auf seine distale Position 130 gefedert gelagert. Zum Auslösen einer Injektion muss das Bedienelement 106 in proximale Richtung 141 bewegt werden. Am Bedienelement 106 ist eine proximale Stirnseite 135 vorgesehen, die mit einer distalen Stirnseite 136 des oberen Gehäuseteils 103 zusammenwirkt und einen Anschlag für die proximale Position 131 des Bedienelements 106 bildet. Die proximale Position 131 des Bedienelements 106 ist in den Fig. 40 bis 42 gezeigt. Durch das Sichtfenster 119 ist bei der gezeigten Position des Einstellrings 115 eine zusätzliche Dosis von null abzulesen. Durch das Sichtfenster 107 und die Öffnung 125 ist vor Beginn der Injektion auch am Außenumfang des Dosierorgans 121 ein Wert von null zu lesen. Durch die Öffnung 125 ist dabei jeweils die bereits ausgepresste Menge an Injektionsflüssigkeit abzulesen.

Durch die Bewegung des Bedienelements 106 in seine in den Figuren 40 bis 42 gezeigte proximale Position 131 wurde die Kupplung 124 geöffnet. Das Bedienelement 106 besitzt Raststege 154, die in der distalen Position 130 des Bedienelements 106 eine drehfeste Verbindung zum Mitnehmer 122 herstellen. In der proximalen Position 131 des Bedienelements 106 sind die Raststege 154 in einem Freiraum 153 des Mitnehmers 122 angeordnet und lassen eine Drehung des Mitnehmers 121 gegenüber dem Bedienelement 106 und damit auch gegenüber dem Gehäuse 102 zu. Die Injektionsfeder 109 wirkt in proximale Richtung auf die Injektionshülse 120. Die Injektionshülse 120 ist in Richtung der Längsmittelachse 150 beweglich, aber drehfest im Gehäuse 102 geführt. Die Injektionshülse 120 besitzt eine proximale Anschlagfläche 147. Am Anschlagelement 116 ist ein distaler Endanschlag 123 ausgebildet, der mit der Anschlagfläche 147 zusammenwirkt und die distale Endlage der Injektionshülse 120 festlegt. Aufgrund der Kopplung der Injektionshülse 120 über das Dosierorgan 121 mit dem Dosierkolben 111 legt der Endanschlag 123 die Endlage des Dosierkolbens 111 indirekt über die Endlage der Injektionshülse 120 fest. Bei der in den Fig. 40 bis 42 eingestellten zusätzlichen Dosis von null Einheiten besitzt die Anschlagfläche 147 zum Endanschlag 123 einen Abstand d. Die proximale Stirnseite 142 des Einstellrings 115 besitzt zur proximalen Stirnseite 140 des oberen Gehäuseteils 103 in dieser Position einen Abstand f. Wie Fig. 42 auch zeigt, sind die Gehäuseteile 103 und 104 über eine Schnappverbindung 143 miteinander verbunden. Die Schnappverbindung 143 umfasst einen nach innen ragenden Schnapprand 144 des oberen Gehäuseteils 103, der in eine Schnappvertiefung 145 des unteren Gehäuseteils 104 ragt. Auch eine andere Verbindung der beiden Gehäuseteile 103 und 104 kann jedoch vorteilhaft sein.

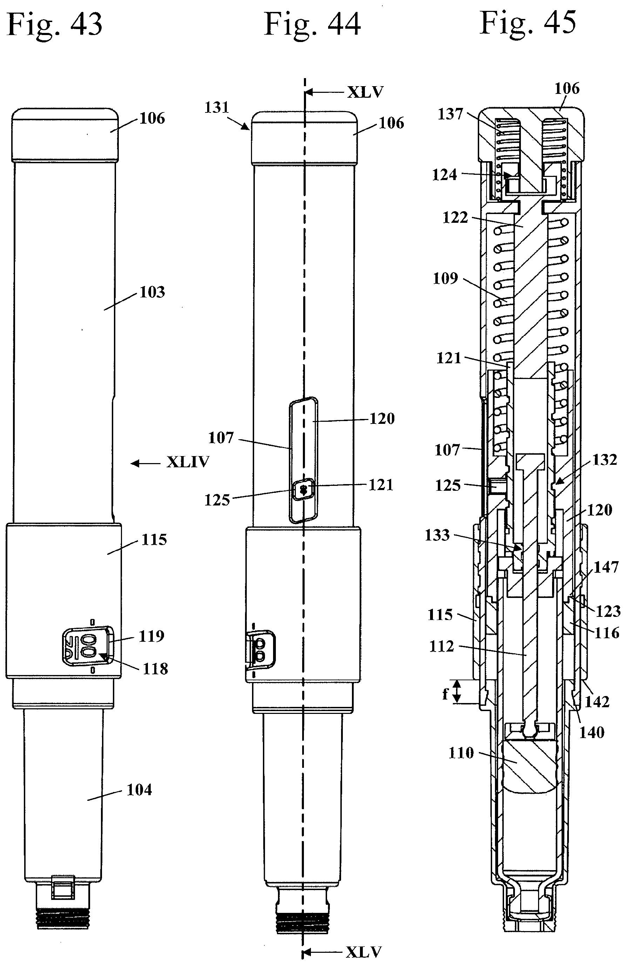

Nachdem die Kupplung 124 geöffnet ist, kann die Injektionsfeder 109 die Injektionshülse 120 in distale Richtung bewegen. Aufgrund der Gewindeverbindung 132 dreht sich dadurch das Dosierorgan 121. Das Dosierorgan 121 kann sich jedoch nicht in axialer Richtung bewegen. Die Gewindeverbindung 133 bewirkt aufgrund der drehfesten Lagerung des Dosierkolbens 111 im Gehäuse 102 eine Bewegung des Dosierkolbens 111 in proximale Richtung, die zum Auspressen von Injektionsflüssigkeit aus dem Behälter 105 führt. Die Injektion ist beendet, wenn die Anschlagfläche 147 am Endanschlag 123 anliegt. Diese Position ist in den Fig. 43 bis 45 gezeigt. Der Einstellring 115 hat seine Position beim Auspressen der Injektionsflüssigkeit nicht verändert. Die Stirnseiten 140 und 142 besitzen weiterhin den Abstand f zueinander. Aufgrund der Bewegung der Injektionshülse 120 in proximale Richtung hat sich die Öffnung 125 gegenüber dem Dosierorgan 121 bewegt, so dass durch die Öffnung 125 nun die ausgepresste Menge an Injektionsflüssigkeit ablesbar ist. Durch das Sichtfenster 119 im Einstellring 115 ist die zusätzlich eingestellte Menge von auszupressender Injektionsflüssigkeit von null Einheiten ablesbar.

Wenn der Bediener das Bedienelement 106 während der Injektion loslässt, wird das Bedienelement 106 in seine distale Position 130 gedrückt, weil es von der Betätigungsfeder 137 in distale Richtung vorgespannt ist. Dadurch wird die Kupplung 124 geschlossen und die Injektion gestoppt. Durch erneutes Drücken des Bedienelements 106 in proximale Richtung 141 kann der Injektionsvorgang fortgesetzt werden.

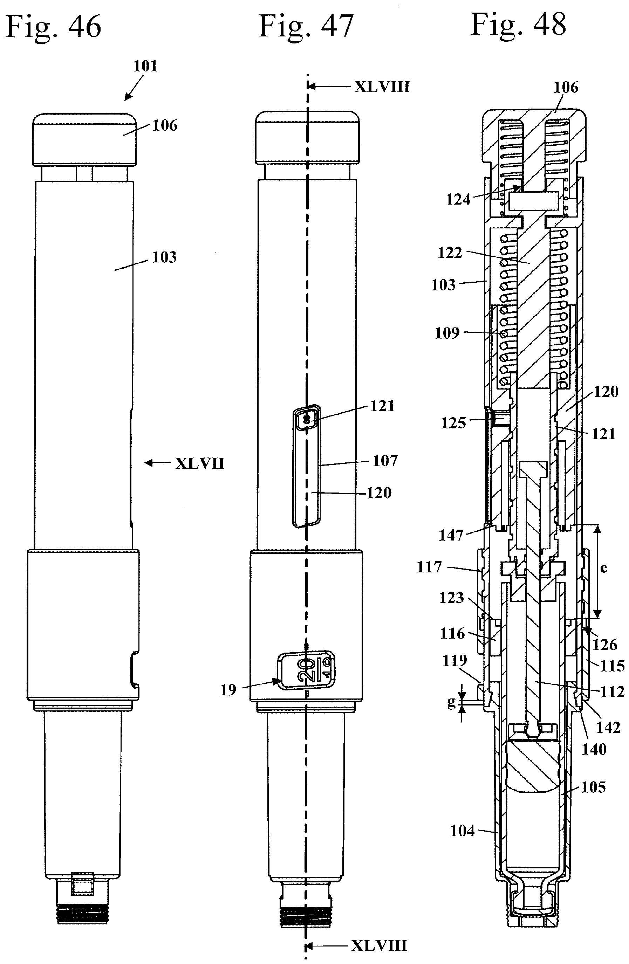

Die Fig. 46 bis 48 zeigen das Injektionsgerät 101 mit der maximal einstellbaren Menge von zwanzig zusätzlichen Einheiten an Injektionsflüssigkeit. Zur Einstellung dieser Menge von Injektionsflüssigkeit wurde der Einstellring 115 gedreht und hat sich aufgrund der Gewindeverbindung 117 in proximale Richtung bewegt. Aufgrund der Koppeleinrichtung 126 wurde das Anschlagelement 116 in proximale Richtung mitgenommen. Die Anschlagfläche 147 besitzt bei dieser eingestellten Dosis zum Endanschlag 123 einen Abstand e, der deutlich größer als der Abstand d (Fig. 42) bei niedrigerer Dosis ist. Der Abstand g der Stirnseiten 142 und 140 von Einstellring 115 und oberem Gehäuseteil 103 ist deutlich geringer als der Abstand f bei der geringeren Dosis (Fig. 42).

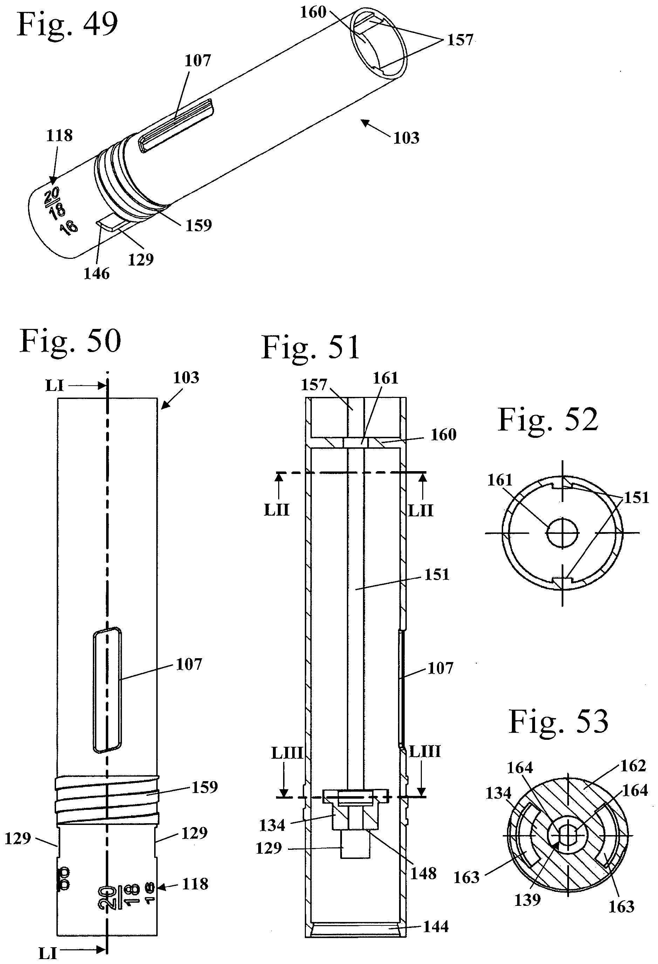

Die Fig. 49 bis 53 zeigen das obere Gehäuseteil 103 im Einzelnen. Das obere Gehäuseteil 103 besitzt zwei einander gegenüberliegend angeordnete Öffnungen 129, durch die die Koppeleinrichtung 126 (Fig. 48) aus dem Inneren des oberen Gehäuseteils 103 zum Einstellring 115 ragen kann. Wie Fig. 49 und Fig. 51 zeigen, bilden die Öffnungen 129 mit ihrer proximalen Stirnseite einen ersten, proximalen Anschlag 146, der die maximale auszupressende Menge an Injektionsflüssigkeit festlegt, und mit ihrer distalen Stirnseite einen distalen, zweiten Anschlag 148, der in Fig. 51 gezeigt ist und der die minimale auszupressende Menge an Injektionsflüssigkeit festlegt. Die Zwischenwand 160 besitzt eine mittige Öffnung 161, durch die der Mitnehmer 122 ragt. Das obere Gehäuseteil 103 trägt ein Außengewinde 159, das Teil der Gewindeverbindung 117 ist. Wie Fig. 49 zeigt, besitzt das obere Gehäuseteil 103 an der distalen Seite der Zwischenwand 160 Führungsstege 157, die zur drehfesten Verbindung des Bedienelements 106 mit dem oberen Gehäuseteil 103 dienen. Die Führungsstege 157 ragen hierzu in die Führungsnuten 158 des Bedienelements 106 (Fig. 37). An der proximalen Seite der Zwischenwand 160 sind zwei einander gegenüberliegend angeordnete Führungsstege 151 vorgesehen (Fig. 51 und 52), die im Ausführungsbeispiel in Verlängerung der Stege 157 angeordnet sind und die zur drehfesten Verbindung mit der Injektionshülse 120 dienen. Die Fig. 51 und 53 zeigen die Gestaltung des Lagerabschnitts 134 im Einzelnen. Der Lagerabschnitt 134 ist an einer Zwischenwand 162 ausgebildet, die seitliche Öffnungen 163 für die Injektionshülse 120 sowie die mittige Führung 139 für die Kolbenstange 112 des Dosierkolbens 111 aufweist. Die Führung 139 besitzt einander gegenüberliegend zwei Abflachungen 164, die zur drehfesten Lagerung der Kolbenstange 112 dienen. Dadurch ist die Kolbenstange 112 drehfest im Gehäuse 102 gelagert.

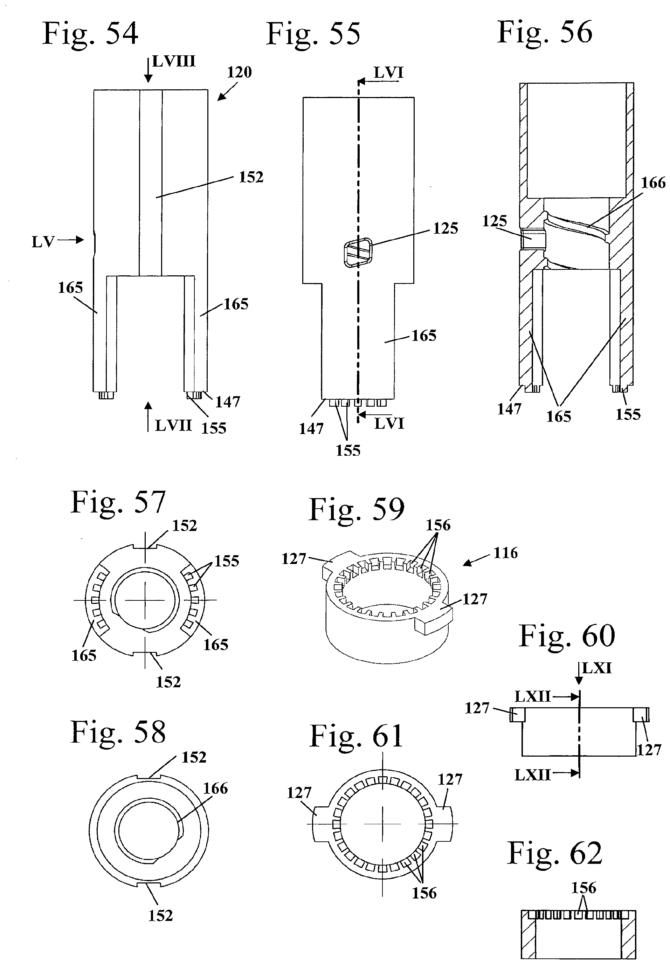

Die Fig. 54 bis 58 zeigen die Injektionshülse 120 im Einzelnen. Die Injektionshülse 120 besitzt an ihrem Außenumfang zwei Führungsnuten 152 (Fig. 55 und 58), in die die Führungsstege 151 des oberen Gehäuseteils 103 zur drehfesten Verbindung ragen. Die Injektionshülse 120 besitzt zwei in proximale Richtung ragende Arme 165, an deren Stirnseiten jeweils ein Abschnitt der Anschlagfläche 147 ausgebildet ist. Im Ausführungsbeispiel ragt aus der Anschlagfläche 147 eine Vielzahl von Zapfen 155, die in entsprechende Vertiefungen 156 des Anschlagelements 116 ragen (Fig. 59). Wie Fig. 56 zeigt, besitzt die Injektionshülse 120 an ihrer Innenseite ein Innengewinde 166, das Teil der Gewindeverbindung 132 ist.

In den Fig. 59 bis 62 ist das Anschlagelement 116 gezeigt. Das Anschlagelement 116 besitzt an seiner distalen Seite zwei einander gegenüberliegende, radial nach außen ragende Stege 127, die durch die Öffnungen 129 des oberen Gehäuseteils 103 ragen und Teil der Koppeleinrichtung 126 sind. Die Stege 127 bilden mit den Längsseiten der Öffnungen 129 gleichzeitig eine Verdrehsicherung für das Anschlagelement 116.

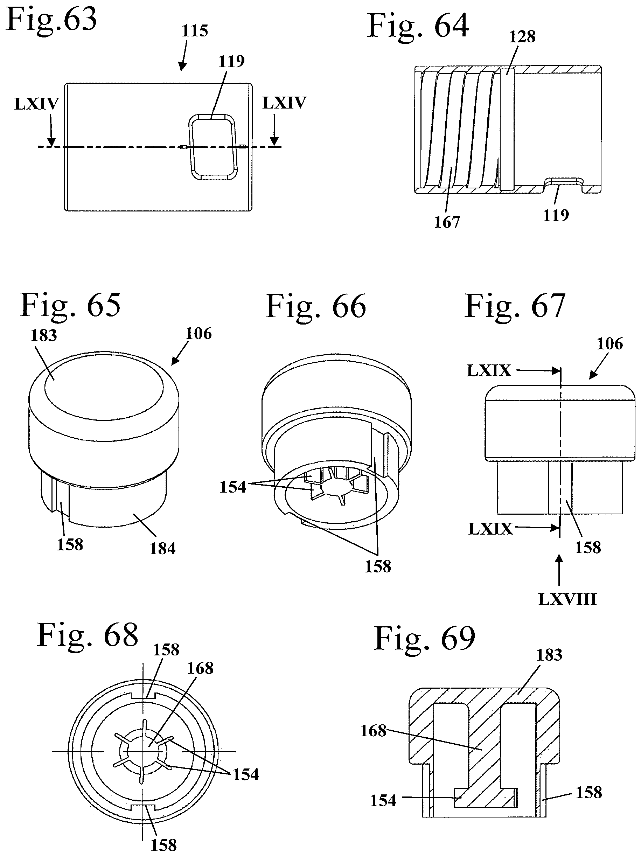

In den Fig. 63 und 64 ist der Einstellring 115 gezeigt. Der Einstellring 115 besitzt an seiner distalen, in den Figuren 63 und 64 links angeordneten Seite ein Innengewinde 167, das mit dem Außengewinde 159 des oberen Gehäuseteils 103 die Gewindeverbindung 117 bildet. An der proximalen Seite des Innengewindes 167 ist eine Führung 128 ausgebildet, die als umlaufende Nut ausgebildet ist und in die Stege 127 des Anschlagelements 116 zur Kopplung der axialen Position von Einstellring 115 und Anschlagelement 116 ragen. Dabei ist vorteilhaft eine weitgehend spielfreie Kopplung vorgesehen, so dass eine genaue Einstellung der auszupressenden Menge an Injektionsflüssigkeit möglich ist.

Die Fig. 65 bis 69 zeigen das Bedienelement 106. Das Bedienelement 106 besitzt einen Betätigungsabschnitt 183 sowie einen Hülsenabschnitt 184, dessen Außendurchmesser geringfügig kleiner als der Innendurchmesser des oberen Gehäuseteils 103 ist. Dadurch kann der Hülsenabschnitt 184 in das obere Gehäuseteil 103 gedrückt werden. Der Hülsenabschnitt 184 trägt an seinem Außenumfang die Führungsnuten 158, in die die Führungsstege 157 des oberen Gehäuseteils 103 zur drehfesten Fixierung des Bedienelements 106 ragen. Vom Betätigungsabschnitt 183 erstreckt sich ein Steg 168 in proximale Richtung. Der Steg 168 trägt an seinem Ende die radial nach außen ragenden Raststege 154, die einen Teil der Kupplung 124 bilden.

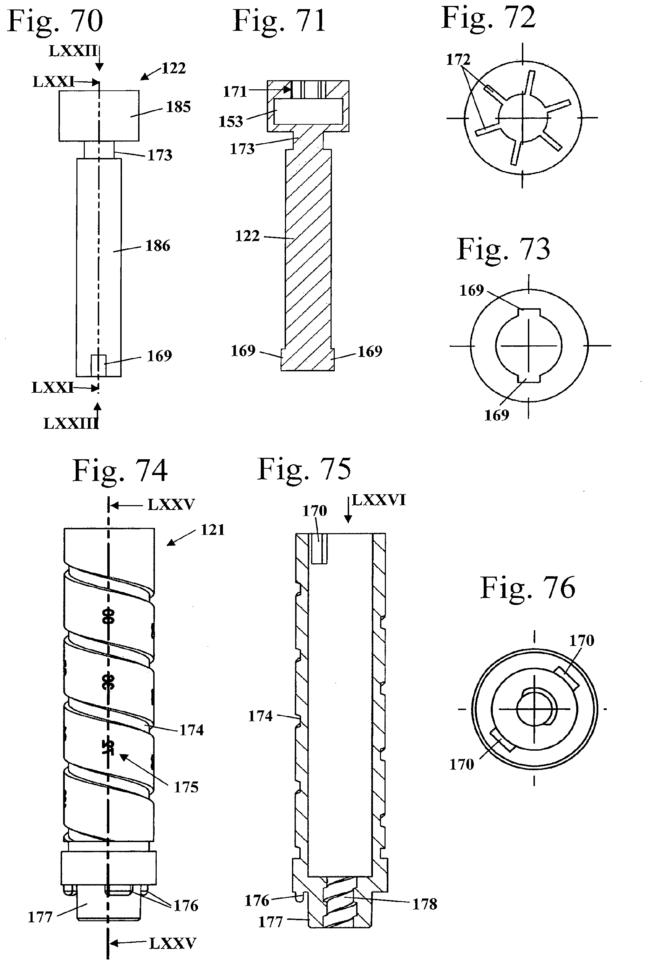

Die Fig. 70 bis 73 zeigen den Mitnehmer 122 im Einzelnen. Der Mitnehmer 122 besitzt einen distalen Abschnitt 185, der über einen Lagerabschnitt 173 von einem proximalen Abschnitt 186 getrennt ist. Mit dem Lagerabschnitt 173 ist der Mitnehmer 122 in der Zwischenwand 160 des oberen Gehäuseteils 103 gelagert. Zur Montage ist der Mitnehmer 122 vorteilhaft mehrteilig ausgebildet. Der distale Abschnitt 185 besitzt einen Koppelabschnitt 171, der, wie Fig. 72 zeigt, eine Vielzahl von Nuten 172 aufweist. Bei geschlossener Kupplung 124 ragen die Raststege 154 des Bedienelements 106 in die Nuten 172 und stellen dadurch eine drehfeste Verbindung zwischen dem Bedienelement 106 und dem Mitnehmer 122 her. An der proximalen Seite des Koppelabschnitts 171 erstreckt sich der Freiraum 153. An seinem distalen Ende trägt der Mitnehmer 122 zwei einander gegenüberliegend angeordnete Verbindungszapfen 169, die zur drehfesten Verbindung mit dem Dosierorgan 121 dienen.

Das Dosierorgan 121 ist in den Fig. 74 bis 76 gezeigt. Das Dosierorgan 121 ist hülsenförmig ausgebildet und trägt an seinem Außenumfang ein Außengewinde 174, das mit dem Innengewinde 166 der Injektionshülse 120 die Gewindeverbindung 132 bildet. An seinem Außenumfang trägt das Dosierorgan 121 außerdem eine Skala 175, die durch die Öffnung 125 sichtbar ist und die die ausgepresste Menge an Injektionsflüssigkeit anzeigt. An der proximalen Seite des Dosierorgans 121 ist ein Lagerzapfen 177 angeordnet, mit dem das Dosierorgan 121 im Lagerabschnitt 134 drehbar gelagert ist. Benachbart zum Lagerzapfen 177 besitzt das Dosierorgan 121 mehrere Vorsprünge 176, an denen das Dosierorgan 121 am Lagerabschnitt 134 aufliegt und die zur Verringerung der Reibung dienen. Wie Fig. 75 zeigt, besitzt das Dosierorgan 121 an seinem distalen Ende an der Innenseite ausgebildete Verbindungsnuten 170, die auf die Verbindungszapfen 169 des Mitnehmers 122 angepasst ist und mit diesen die drehfeste Verbindung von Mitnehmer 122 und Dosierorgan 121 bilden. Am proximalen Ende des Dosierorgans 121 ist innerhalb des Lagerzapfens 177 ein Innengewinde 178 vorgesehen, das Teil der Gewindeverbindung 133 ist.

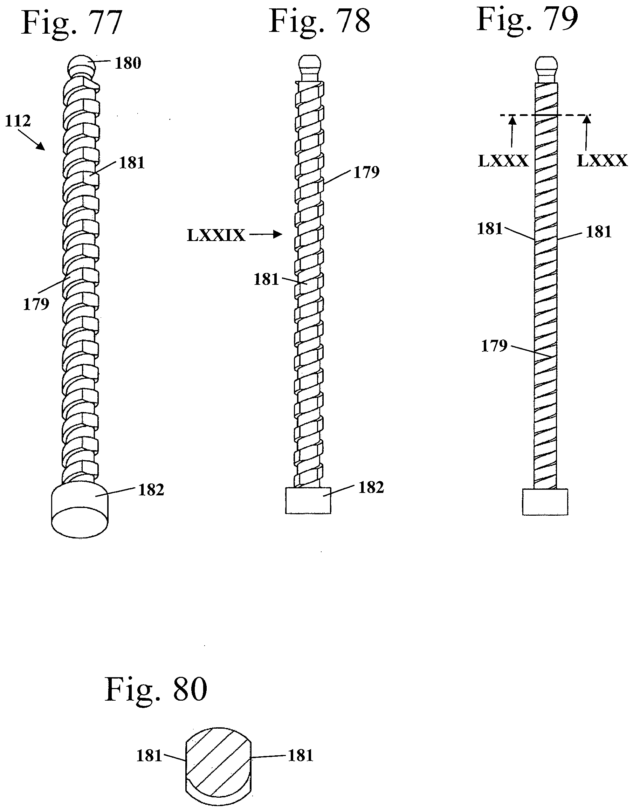

Die Fig. 77 bis 80 zeigen die Kolbenstange 112. Die Kolbenstange 112 besitzt ein Außengewinde 179, das mit dem Innengewinde 178 die Gewindeverbindung 133 bildet. An seiner distalen, in den Fig. 77 bis 79 unten gezeigten Seite besitzt die Kolbenstange 112 eine Endscheibe 182, die verhindert, dass sich die Kolbenstange 112 durch das Innengewinde 178 des Dosierorgans 121 schrauben kann. An seiner proximalen Seite weist die Kolbenstange 112 einen Zapfen 180 zur Verbindung mit der Kolbenscheibe 113 auf. Das Außengewinde 179 weist in Längsrichtung verlaufende Abflachungen 181 auf, die auch in den Fig. 78, 79 und 80 gezeigt sind. Die Abflachungen 181 dienen zur drehfesten Verbindung mit dem Gehäuse 102 und liegen an den Abflachungen 164 (Fig. 53) des oberen Gehäuseteils 103 an.

## Patentansprüche

1. Injektionsgerät zum automatischen Auspressen von Injektionsflüssigkeit aus einem Behälter, mit einem Gehäuse (2, 102), mit einer Injektionsfeder (9, 109), die das Auspressen der Injektionsflüssigkeit aus dem Behälter (5, 105) bewirkt, mit einem Dosierkolben (11, 111), der in Richtung einer Längsmittelachse (50, 150) des Injektionsgeräts (1, 101) beweglich gelagert ist und sich beim Auspressen von Injektionsflüssigkeit in proximaler Richtung (41, 141) bewegt, mit einer Einstellvorrichtung (14, 114) zur Einstellung der auszupressenden Menge an Injektionsflüssigkeit, und mit einem Endanschlag (23, 123), der die Endlage des Dosierkolbens (11, 111) festlegt, wobei die Einstellvorrichtung (14, 114) Mittel zur Verstellung der Lage des Endanschlags (23, 123) besitzt,
**dadurch gekennzeichnet, dass** das Injektionsgerät (101) eine drehfest im Gehäuse (102) gelagerte Injektionshülse (120) besitzt, die über eine erste Gewindeverbindung (132) mit einem drehbar und axial unverschiebbar im Gehäuse (102) gelagerten Dosierorgan (121) verbunden ist, und dass das Dosierorgan (121) über eine zweite Gewindeverbindung (133) mit dem Dosierkolben (111) verbunden ist.

2. Injektionsgerät nach Anspruch 1,
**dadurch gekennzeichnet, dass** die Einstellvorrichtung (14, 114) die Lage des Endanschlags (23, 123) in Richtung einer Längsmittelachse (50, 150) des Injektionsgeräts (1, 100) verstellt.

3. Injektionsgerät nach Anspruch 1 oder 2
**dadurch gekennzeichnet, dass** die Einstellvorrichtung (14, 114) ein im Gehäuse (2, 102) angeordnetes Anschlagelement (16, 116) und einen mindestens teilweise auf die Gehäuseaußenseite ragenden, vom Bediener zu betätigenden Einstellring (16, 116) umfasst.

4. Injektionsgerät nach Anspruch 3,
**dadurch gekennzeichnet, dass** der Einstellring (15, 115) drehbar gelagert ist.

5. Injektionsgerät nach Anspruch 4,
**dadurch gekennzeichnet, dass** der Einstellring (15, 115) über eine Gewindeverbindung (17, 117) mit dem Gehäuse (2, 102) verbunden ist und eine Drehung des Einstellrings (15, 115) eine Bewegung des Einstellrings (15, 115) in Richtung der Längsmittelachse (50, 150) des Injektionsgeräts (1, 101) bewirkt.

6. Injektionsgerät nach einem der Ansprüche 3 bis 5,
**dadurch gekennzeichnet, dass** das Injektionsgerät (1, 100) eine Koppeleinrichtung (26, 126) umfasst, die die axiale Lage des Anschlagelements (16, 116) an die axiale Lage des Einstellrings (15, 115) koppelt.

7. Injektionsgerät nach Anspruch 6,
**dadurch gekennzeichnet, dass** die Koppeleinrichtung (26, 126) mindestens ein Koppelelement umfasst, das durch eine Öffnung (29, 129) im Gehäuse (2, 102) in eine Führung (28, 128) der Koppeleinrichtung (26, 126) ragt.

8. Injektionsgerät nach Anspruch 6 oder 7,
**dadurch gekennzeichnet, dass** das Anschlagelement (16, 116) im Gehäuse (2, 102) drehfest geführt ist.

9. Injektionsgerät nach Anspruch 8,
**dadurch gekennzeichnet, dass** das mindestens eine Koppelelement mit der Öffnung (29, 129) im Gehäuse (2, 102) die drehfeste Führung des Anschlagelements (16, 116) bewirkt.

10. Injektionsgerät nach einem der Ansprüche 1 bis 9,
**dadurch gekennzeichnet, dass** der Dosierkolben (11) einen Rand (22) besitzt, der mit dem Endanschlag (23) zusammenwirkt.

11. Injektionsgerät nach Anspruch 10,
**dadurch gekennzeichnet, dass** sich die Injektionsfeder (9) an der distalen Seite des Rands (22) abstützt.

12. Injektionsgerät nach einem der Ansprüche 1 bis 11,
**dadurch gekennzeichnet, dass** die Injektionshülse (120) eine Anschlagfläche (147) besitzt, die mit dem Endanschlag (123) zusammenwirkt.

13. Injektionsgerät nach einem der Ansprüche 1 bis 12,
**dadurch gekennzeichnet, dass** sich die Injektionsfeder (109) mit ihrem proximalen Ende an der Injektionshülse (120) abstützt.

## Claims

1. Injection device for automatically squeezing injection liquid from a container, with a housing (2, 102), with an injection spring (9, 109) causing the squeezing of the injection liquid from the container (5, 105), with a metering piston (11, 111), which is movably mounted in the direction of a longitudinal central axis (50, 150) of the injection device (1, 101) and moves in a proximal direction (41, 141) when squeezing out injection liquid, with a setting device (14, 114) for setting the quantity of injection liquid to be squeezed out, and with an end stop (23, 123) which determines the end position of the metering piston (11, 111), the setting device (14, 114) having means for adjusting the position of the end stop (23, 123), **characterised in that** the injection device (101) has an injection sleeve (120), which is non-rotatably mounted in the housing (102) and connected via a first threaded connection (132) to a metering member (121) mounted rotatably and in an axially fixed manner in the housing (102), and **in that** the metering member (121) is connected to the metering piston (111) via a second threaded connection (133).

2. Injection device according to claim 1,
**characterised in that** the setting device (14, 114) adjusts the position of the end stop (23, 123) in the direction of a longitudinal central axis (50, 150) of the injection device (1, 101).

3. Injection device according to claim 1 or 2,
**characterised in that** the setting device (14, 114) comprises a stop element (16, 116) located in the housing (2, 102) and a setting ring (16, 116), which at least partially projects to the outside of the housing and is configured for operation by the operator.

4. Injection device according to claim 3,
**characterised in that** the setting ring (15, 115) is mounted rotatably.

5. Injection device according to claim 4,
**characterised in that** the setting ring (15, 115) is connected to the housing (2, 102) via a threaded connection (17, 117) and a rotation of the setting ring (15, 115) causes a movement of the setting ring (15, 115) in the direction of the longitudinal central axis (50, 150) of the injection device (1, 101).

6. Injection device according to any of claims 3 to 5,
**characterised in that** the injection device (1, 101) comprises a coupling installation (26, 126), which couples the axial position of the stop element (16, 116) to the axial position of the setting ring (15, 115).

7. Injection device according to claim 6,
**characterised in that** the coupling installation (26, 126) comprises at least one coupling element which projects through an opening (29, 129) in the housing (2, 102) into a guide (28, 128) of the coupling installation (26, 126).

8. Injection device according to claim 6 or 7,
**characterised in that** the stop element (16, 116) is non-rotatably guided in the housing (2, 102).

9. Injection device according to claim 8,
**characterised in that** the at least one coupling element causes the non-rotatable guidance of the stop element (16, 116) with the opening (29, 129) in the housing (2, 102).

10. Injection device according to any of claims 1 to 9,
**characterised in that** the metering piston (11) has an edge (22), which interacts with the end stop (23).

11. Injection device according to claim 10,
**characterised in that** the injection spring (9) is supported on the distal side of the edge (22).

12. Injection device according to any of claims 1 to 11,
**characterised in that** the injection sleeve (120) has a stop surface (147) interacting with the end stop (123).

13. Injection device according to any of claims 1 to 12,
**characterised in that** the injection spring (109) is supported on the injection sleeve (120) by its proximal end.

## Revendications

1. Appareil d'injection pour l'expulsion automatique d'un liquide d'injection d'un contenant pourvu d'un logement (2, 102), d'un ressort d'injection (9, 109) qui provoque l'expulsion du liquide d'injection du contenant (5, 105), d'un piston de dosage (11, 111) qui est monté mobile en direction d'un axe longitudinal médian (50, 150) de l'appareil d'injection (1, 101) et se déplace dans la direction proximale (41, 141) lors de l'expulsion du liquide d'injection, d'un dispositif de réglage (14, 114) destiné à régler la quantité de liquide d'injection à expulser, et d'une butée de fin de course (23, 123) qui définit la position finale du piston de dosage (11, 111), dans lequel le dispositif de réglage (14, 114) possède des moyens servant à régler la position de la butée de fin de course (23, 123),
**caractérisé en ce que** l'appareil d'injection (101) possède un manchon d'injection (120) monté solidaire en rotation dans le logement (102) et qui est relié à un organe de dosage (121) monté dans le logement (102) à rotation et de manière à ne pas pouvoir coulisser axialement par l'intermédiaire d'une première liaison filetée (132), et **en ce que** l'organe de dosage (121) est relié au piston de dosage (111) par l'intermédiaire d'une deuxième liaison filetée (133).

2. Appareil d'injection selon la revendication 1,
**caractérisé en ce que** le dispositif de réglage (14, 114) règle la position de la butée de fin de course (23, 123) en direction d'un axe longitudinal médian (50, 150) de l'appareil d'injection (1, 100).

3. Appareil d'injection selon la revendication 1 ou 2,
**caractérisé en ce que** le dispositif de réglage (14, 114) comporte un élément de butée (16, 116) agencé dans le logement (2, 102) et une bague de réglage (16, 116) faisant saillie au moins en partie de la face extérieure du logement et destinée à être actionnée par l'utilisateur.

4. Appareil d'injection selon la revendication 3,
**caractérisé en ce que** la bague de réglage (15, 115) est montée à rotation.

5. Appareil d'injection selon la revendication 4,
**caractérisé en ce que** la bague de réglage (15, 115) est reliée au logement (2, 102) par l'intermédiaire d'une liaison filetée (17, 117) et une rotation de la bague de réglage (15, 115) provoque un déplacement de la bague de réglage (15, 115) en direction de l'axe longitudinal médian (50, 150) de l'appareil d'injection (1, 101).

6. Appareil d'injection selon l'une des revendications 3 à 5,
**caractérisé en ce que** l'appareil d'injection (1, 100) comporte un dispositif d'accouplement (26, 126), qui accouple la position axiale de l'élément de butée (16, 116) à la position axiale de la bague de réglage (15, 115).

7. Appareil d'injection selon la revendication 6,
**caractérisé en ce que** le dispositif d'accouplement (26, 126) comporte au moins un élément d'accouplement qui fait saillie dans un guidage (28, 128) du dispositif d'accouplement (26, 126) à travers une ouverture (29, 129) dans le logement (2, 102).

8. Appareil d'injection selon la revendication 6 ou 7,
**caractérisé en ce que** l'élément de butée (16, 116) est guidé de manière solidaire en rotation dans le logement (2, 102).

9. Appareil d'injection selon la revendication 8,
**caractérisé en ce que** l'au moins un élément d'accouplement provoque avec l'ouverture (29, 129) dans le logement (2, 102) le guidage solidaire en rotation de l'élément de butée (16, 116).

10. Appareil d'injection selon l'une des revendications 1 à 9,
**caractérisé en ce que** le piston de dosage (11) possède un bord (22) qui coopère avec la butée de fin de course (23).

11. Appareil d'injection selon la revendication 10,
**caractérisé en ce que** le ressort d'injection (9) s'appuie sur la face distale du bord (22).

12. Appareil d'injection selon l'une des revendications 1 à 11,
**caractérisé en ce que** le manchon d'injection (120) possède une surface de butée (147), qui coopère avec la butée de fin de course (123).

13. Appareil d'injection selon l'une des revendications 1 à 12,
**caractérisé en ce que** le ressort d'injection (109) s'appuie sur le manchon d'injection (120) par son extrémité proximale.
